(19) Europäisches Patentamt
European Patent Office
Office européen des brevets

(11) **EP 1 041 384 B1**

(12) **EUROPEAN PATENT SPECIFICATION**

(45) Date of publication and mention
of the grant of the patent:
**14.05.2008 Bulletin 2008/20**

(51) Int Cl.:
*G01N 33/44* *(2006.01)*      *G01N 11/16* *(2006.01)*

(21) Application number: **00201069.2**

(22) Date of filing: **23.03.2000**

(54) **Method for determining the behaviour of a viscoelastic material**

Verfahren zur Bestimmung des Verhaltens eines viskoelastischen Materials

Procédé de détermination du comportement d'un matériau viscoélastique

(84) Designated Contracting States:
**AT BE CH CY DE DK ES FI FR GB GR IE IT LI LU
MC NL PT SE**

(30) Priority: **29.03.1999 EP 99200987**

(43) Date of publication of application:
**04.10.2000 Bulletin 2000/40**

(73) Proprietor: **Pirelli Tyre S.p.A.**
**20126 Milano (IT)**

(72) Inventors:
• **Brunacci, Antonio**
**2351 Luxembourg (LU)**
• **Nahmias Nanni, Marco**
**20121 Milano (IT)**
• **Serra, Antonio**
**16138 Genova (IT)**

(74) Representative: **Marchi, Massimo et al**
**Marchi & Partners,**
**Via Pirelli, 19**
**20124 Milano (IT)**

(56) References cited:
**EP-A- 0 367 218          EP-A- 0 466 060**
**EP-A- 0 545 728          WO-A-93/09419**
**US-A- 4 667 519          US-A- 5 452 614**
**US-A- 5 784 283**

• **J. SOUSA ET AL.: "Dynamic Properties of Asphalt
Concrete" JOURNAL OF TESTING AND
EVALUATION., vol. 16, no. 4, July 1988 (1988-07),
pages 350-363, XP002114023 PHILADELPHIA,
PA, US ISSN: 0090-3973**

**Description**

The present invention relates to a method for determining the behaviour of a viscoelastic material.

It also relates to a method for improving the storage of experimental curves, a method for determining at least one viscoelastic material having predetermined characteristics.

Among the properties of viscoelastic materials, those which are mainly associated with their use are the dynamic properties, or the responses to the application of deformations.

At present, in order to be able to select, at the time of use, the material which best satisfies the final dynamic requirements of a desired product, it is necessary to know the dynamic properties of a large number of materials and, therefore, to have first carried out numerous experimental measurements in order to characterize them.

These dynamic properties, however, depend on the temperature and, in the case of some materials, such as for example polymers containing reinforcing fillers or asphalts, also on the deformation applied. Consequently, the characterization of each material requires the acquisition of a large number of experimental values according to variation in the temperature and the deformation applied.

For example, in order to determine the dynamic properties of these materials, generally experimental measurements of the force following deformation cycles in a predetermined temperature range are performed.

Moreover, the storage of the abovementioned data requires a large amount of space or memory depending on whether it is stored on a paper medium or electronic medium. In the case of storage on a paper medium, moreover, a very long time is needed in order to check whether, with the stored experimental data, it is possible to identify a product having the required characteristics, at a certain temperature.

Another drawback inherent in the known methods is that it is possible to identify the product having the required characteristics only if experimental have been performed at the temperature concerned at the moment when said identification is performed.

US-A-4 667 519 discloses an apparatus for generating data for measuring rheological/viscoelastic properties of a curing rubber sample, the apparatus comprising:

- stressing means having a variable displacement including a maximum displacement for causing variable stress in the curing rubber sample;
- measuring means for generating measurements of instantaneous stress in the rubber sample when curing, on command;
- means for sensing maximum displacement and generating both a maximum displacement signal and a time interval passage signal, the time interval passage signal occurring upon passage of a time interval after maximum displacement;
- controlling means for receiving and acting upon the maximum displacement signal and the time interval passage signal to command the measuring means.

US-A-5 452 614 discloses a dynamic viscoelasticity apparatus comprising:

- means for holding a sample;
- means for applying a sine wave strain deformation through said means for holding the sample and for providing a signal representative of the sine wave strain deformation;
- detector means for producing a signal representative of a response by the sample to the strain deformation applied by said applying means;
- means for varying the temperature of the sample;
- first analog-to-digital converter means for converting the signal produced by said detector means into a corresponding digital deformation signal;
- second analog-to-digital converter means for converting the signal produced by said means for applying a sine wave strain deformation into a corresponding digital strain signal;
- memory means for storing the digital deformation and strain signals from said first and second analog-to-digital converter means within predetermined periods of the sine wave strain deformation;
- calculator means for performing Fourier transformation calculation using digital deformation and strain signals in said memory means and for calculating the result of the Fourier transformation calculation to correct for creep based on the change in the signal produced by said detector means during one period of the sine wave and for performing calculations of sample dynamic viscoelasticity based on the result of the Fourier transformation calculation; and
- memory clear means for clearing the digital signals stored in said memory means just after transferring the signals of the memory means to the calculator means so as to prepare said memory means for storing new signals from the first and second analog-to-digital converter means.

The inventors of the present invention, therefore, have considered the problem of simplifying the characterization, storage and selection processes which are currently used.

More precisely, considering the experimental curves a dynamic parameter P as a function of a deformation q applied to a generic test piece of viscoelastic material at a temperature T, the inventors of the present invention have surprisingly that these experimental curves are referable to a summation of exponentials of the type:

$$P(q,T) = a*T^2 + b*T + c + \sum_{i=1}^{\infty}\left(d_i*T^2 + e_i*T + f_i\right)*e^{-\frac{q}{q_i}} \qquad (I)$$

where

- q is the deformation expressed in %;
- T is the temperature expressed in Kelvin;
- $P(q,T)$ is a dynamic parameter preferably selected from the group comprising the elastic modulus, the viscous modulus, the complex modulus and the loss factor;
- a, b, c are characteristic constants which depend on the type of viscoelastic material and the type of dynamic parameter considered;
- $d_i$, $e_i$, $f_i$ are characteristic constants which depend on the effect of the temperature on the dynamic parameter P and on the ith characteristic deformation;
- $q_i$ is the characteristic deformation at the ith exponential.

The inventors, moreover, have realized that, in order to obtain good approximation of said experimental curves, it is sufficient to perform the abovementioned summation (I) as far as the second term. In such a case, therefore, there are only 11 parameters to be determined for each dynamic parameter P of each viscoelastic material, namely, a, b, c, $q_1$, $q_2$, $d_1$, $e_1$, $f_1$, $d_2$, $e_2$ and $f_2$.

In the case where, on the other hand, it is desired to obtain a greater accuracy, it is sufficient to perform the abovementioned summation (I) as far as the higher term, which ensures the desired accuracy. Obviously, in this case, the number of parameters which must be determined will gradually increase.

Therefore, even though the description which follows is mainly based on carrying out the summation as far as the second term, the person skilled in the art will not have any difficulty in determining the required parameters and in carrying out the abovementioned summation (I) up to any term higher than the second term which ensures the desired accuracy.

For example, in the case of the experimental curves for the elastic modulus G', measured at different temperature values T, as a function of a torsion $\gamma$ applied to a cylindrical test piece consisting of a compound for a tyre containing a reinforcing filler, the inventors have found that these experimental curves are referable, according to the present invention, to the relation:

$$G(\gamma,T) = a*T^2 + b*T + c + \left(d_1*T^2 + e_1*T + f_1\right)*e^{-\frac{\gamma}{\gamma_1}} + \left(d_2*T^2 + e_2*T + f_2\right)*e^{-\frac{\gamma}{\gamma_2}}$$

Furthermore, investigating also the experimental curves of the complex modulus G*, viscous modulus G"

(where $\left| G * \right| = \sqrt{\left(G'\right)^2 + \left(G''\right)^2}$ )

and the loss factor $\tan\delta$ (where $\tan\delta = G''/G'$) as a function of the torsion $\gamma$, applied to the test piece, and of the temperature T, the inventors have surprisingly found that they may also be represented by the abovementioned summation of exponentials and that in order to obtain a good approximation, for each of the abovementioned experimental curves, 11 parameters are sufficient, as illustrated above.

Finally, the inventors have even more surprisingly found that the values of the parameters a, b, c, $q_1$, $q_2$, $d_1$, $e_1$, $f_1$, $d_2$, $e_2$ and $f_2$ ,which have been determined on the basis of at least 5 experimental measurements, carried out at each of at least three temperatures $T_x$, $T_y$, $T_z$, of a dynamic parameter P of a viscoelastic material as a function of a deformation q, may be used to determine the progression of said dynamic parameter P as a function of said deformation q also at a temperature $T_w$ different from $T_x$, $T_y$ and $T_z$. Thus, with this method the progression of said dynamic parameter P is determined as a function of said deformation q at a temperature $T_w$ for which no experimental determination was performed.

According to a first aspect thereof, the present invention therefore relates to a method for determining the behaviour of a viscoelastic material at a temperature $T_w$ according to claim 1.

More particularly, during said determination for subsequent approximations of step d)

d1) an arbitrary value for each of said parameters a, b, C, $q_1$, $q_2$, $d_1$, $e_1$, $f_1$, $d_2$, $e_2$, $f_2$ is inserted in said relation (A);

d2) by means of said relation (A) at least 11 values of said parameter P are determined as a function of the values of said deformation q indicated in the abovementioned step c);

d3) the differences between the values, thus determined, of said parameter P and the corresponding values of P indicated in the abovementioned step c) are calculated;

d4) the squares of the abovementioned differences are added together;

d5) the value thus obtained of the sum of the squares of the abovementioned differences is stored;

d6) on the basis of the value obtained for said sum, another value is assigned to each of the parameters a, b, c, $q_1$, $q_2$, $d_1$, $e_1$, $f_1$, $d_2$, $e_2$, $f_2$ so as to reduce this sum of the squares of the differences;

d7) the steps from d2) to d6) are repeated until the value of the sum of the squares of the differences is minimized;

d8) the values of said parameters a, b, c, $q_1$, $q_2$, $d_1$, $e_1$, $f_1$, $d_2$, $e_2$, $f_2$ which minimized the value of said sum are stored.

These latter values, which are inserted in the abovementioned relation (A), generate the curves which best approximate the experimental curves plotted in the abovementioned step b).

Preferably, at least some of said at least 11 values of the deformation q, used in step d2) to determine P by means of the relation (A), are selected from among those used during the preceding step a). More preferably, all said at least 11 values of the deformation q used in step d2) are chosen from among the values of the deformation q used in said step a).

Preferably, in the abovementioned step c), at least two values of said dynamic parameter P and the associated deformation q for each of said experimental curves are chosen. More preferably, in the abovementioned step c), at least three values of said dynamic parameter P and the associated deformation q for each of said experimental curves are chosen.

Typically, said dynamic parameter P is selected from the group comprising the elastic modulus P', the viscous modulus P", the complex modulus P* and the loss factor tanδ of said viscoelastic material. Moreover, said deformation q is selected from the group comprising a torsion γ, a cutting force τ, a tensioning force and a flexing force.

Advantageously, said at least three temperatures $T_x$, $T_y$, $T_z$ are selected in a range of temperatures laying between -55°C and 90°C.

When it is required to obtain greater accuracy, the additional parameters required are determined and the summation (I) is performed:

$$P(q,T) = a*T^2 + b*T + c + \sum_{i=1}^{\infty} \left( d_i * T^2 + e_i * T + f_i \right) * e^{-\frac{q}{q_i}} \qquad (I)$$

where

- q is the deformation expressed in %;
- T is the temperature expressed in Kelvin;
- a, b, c are characteristic constants which depend on the type of viscoelastic material and on the type of dynamic parameter considered;
- $d_i$, $e_i$, $f_i$ are characteristic constants which depend on the effect of the temperature on the dynamic parameter P and on the ith characteristic deformation;
- qi is the characteristic deformation at the ith exponential.

According to a second aspect thereof, the present invention relates to a method for improving the storage of at least three experimental curves, according to claim 5. experimental curves;

c) storing the values of said parameters a, b, c, $q_1$, $q_2$, $d_1$, $e_1$, $f_1$, $d_2$, $e_2$, $f_2$ thus obtained.

As regards the characteristics of said determination for subsequent approximations, said dynamic parameter P, said deformation q and said temperatures $T_x$, $T_y$, $T_z$, reference should be made to that already stated above in connection with the method for determining the behaviour of a viscoelastic material at a temperature $T_w$.

According to a third aspect thereof, the present invention relates to a method for defining, from among a plurality of viscoelastic materials, at least one viscoelastic material having a dynamic parameter according to claim 7.

Preferably, said parameters a, b, c, $q_1$, $q_2$, $d_1$, $e_1$, $f_1$, $d_2$, $e_2$, $f_2$ of the abovementioned step a) were determined in a manner similar to that already described further above in connection with the first and the second aspect of the present invention.

Moreover, as regards the characteristics of said dynamic parameter P, said deformation $q_w$ and said temperature $T_w$, reference should be made to that stated further above in connection with P, q, $T_x$, $T_y$ and $T_z$.

According to the present invention, in order to characterize a dynamic property P as a function of a deformation q of a viscoelastic material at a temperature $T_w$ at which no experimental measurement has been performed, the results of at least 5 experimental measurements, carried out at each of at least three temperatures $T_x$, $T_y$, $T_z$, of said dynamic parameter P as a function of said deformation q are sufficient. In fact, on the basis of these experimental measurements, it is possible to determine values of the abovementioned parameters a, b, c, $q_1$, $q_2$, $d_1$, $e_1$, $f_1$, $d_2$, $e_2$, $f_2$, on the basis of which, inserted in the relation (A), it is possible to obtain the progression of the dynamic parameter P as a function of the deformation q also at the temperature $T_w$, different from $T_x$, $T_y$, and $T_z$, at which experimental measurements have not been performed.

The present invention therefore has the advantage of reducing the number of experimental measurements which must be performed in order to characterize a viscoelastic material.

Moreover, as a result of the present invention, it is possible to select, from among a plurality of viscoelastic materials, that material / those materials for which a preselected dynamic parameter P best approximates a desired value $P_w$ for a preselected deformation $q_w$ at a temperature $T_w$ at which experimental measurements have not been performed. Namely, with the present invention it is possible to define a product having the required characteristics even though experimental measurements at the temperature concerned have not been performed at the moment in which said defining operation is performed.

Obviously, instead of defining a single product having a predetermined value $P_w$ of said predetermined dynamic parameter P, with the invention it is also possible to define classes of materials for which, at a preselected deformation $q_w$ and temperature $T_w$, a dynamic parameter P is greater or less than a given threshold value $P_w$.

The invention therefore simplifies, at the time of use, the process for selection of the viscoelastic material (or viscoelastic materials) which, from among those characterized, best satisfies (or satisfy) the final dynamic requirements of a desired product.

Finally, with the present invention it is possible to improve storage of N measured experimental values of a dynamic parameter P as a function of a deformation q at each of M temperatures T, reducing the number of values to be stored from (2xNxM)+(M) to 11 (in addition to the experimentally measured values of a dynamic parameter P it is also necessary to store the corresponding deformation values N and temperature values M used in the measurement). Since, in order to characterize univocally the dynamic properties of a viscoelastic material, it is necessary to know the behaviour of at least two dynamic parameters as a function of the deformation and the temperature (for example, the elastic modulus P' and the viscous modulus P'') and since, for each dynamic parameter, many measurements ($N \geq 5$, $M \geq 3$) are generally necessary, it is obvious that with the method according to the invention it is possible to reduce considerably the number of values to be stored.

Example 1

60 parts by weight of carbon black, type N234, and vulcanizing agents, vulcanization accelerators, activating agents, anti-ageing agents and conventional plasticizers well known in the manufacture of compounds for treads were added to 100 parts by weight of a compound for tyre treads consisting of a mixture of 70 parts by weight of Styrene Butadiene Rubber (SBR), 20 parts by weight of Butadiene Rubber (BR) and 10 parts by weight of natural rubber (NR). The compound thus obtained was then subjected to a conventional vulcanization treatment based on sulphur at a temperature of 151°C for 30 minutes. Finally, cylindrical test pieces with a diameter of 5 $\pm$ 0.2 mm and height of 6 $\pm$ 0.2 mm were prepared from this compound.

Torsional tests at a frequency of 1 Hz and at temperatures of (-20, -10, 0, 10, 23, 70) $\pm$ 2°C for different values of the torsional angle $\alpha$ applied were carried out on these test pieces. For this purpose a machine known as an Asphalt Analyser made by the company RHEOMETRIC was used, said machine having been set up beforehand for analysing the behaviour of the test piece in a range of deformations comprised between 0.05% and 40%, with logarithm-type scanning of the deformations applied.

With this arrangement, considering that the dependency of the deformation $\gamma$ of the applied torsional angle $\alpha$ is expressed by the following relation:

$$\gamma\ (\%)\ =\ \alpha\ \times\ \frac{R}{h} \times 100$$

where

R and h are the radius and the height of the cylindrical test piece, respectively, the machine provided 29 measured values of the elastic modulus G' - expressed in Pascal (Pa) - as a function of 29 values of the deformation $\gamma$ - expressed in % - for each of the 6 temperature values considered.

These values for the deformation $\gamma$, elastic modulus G' and temperature were used, according to the invention, to determine, for subsequent approximations, values of the parameters a, b, c, $q_1$, $q_2$ $d_1$, $e_1$, $f_1$, $d_2$, $e_2$, $f_2$.

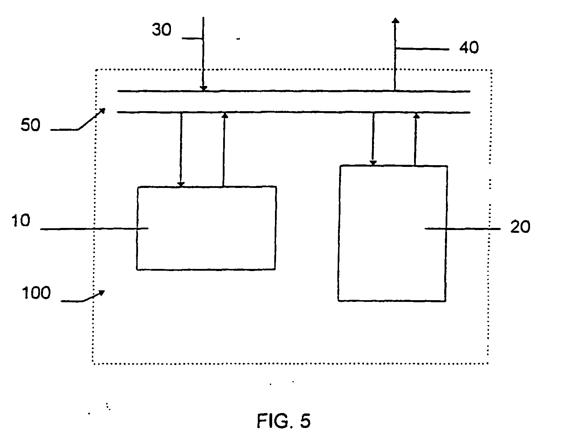

More particularly, these values of the parameters a, b, c, $q_1$, $q_2$, $d_1$, $e_1$, $f_1$, $d_2$, $e_2$, $f_2$ were determined with an apparatus 100 which, according to the invention, comprises (Fig. 5) an input 30, a processing unit 10, a memory 20, an output 40 and connection lines 50.

Via said input 30, said 29 experimentally determined values of said elastic modulus G' as a function of said 29 values of the deformation $\gamma$ at said six temperatures (-20, -10, 0, 10, 23, 70) $\pm$ 2°C were entered. Said processing unit 10 was then activated by a suitable calculator programme so as to:

1) assign an arbitrary value for each of the parameters a, b, c, $q_1$, $q_2$, $d_1$, $e_1$, $f_1$, $d_2$, $e_2$, $f_2$;
2) insert this arbitrary value of the parameters a, b, c, $q_1$, $q_2$, $d_1$, $e_1$, $f_1$, $d_2$, $e_2$, $f_2$, in the abovementioned relation (A);
3) use said relation (A), in which said arbitrary values were inserted, in order to calculate, at each of said six temperatures (-20, - 10, 0, 10, 23, 70) $\pm$ 2°C, twenty-nine values of G' as a function of the abovementioned twenty-nine values of $\gamma$ used in the experimental measurements;
4) calculate the differences between the values of G' thus calculated and the corresponding values measured experimentally;
5) add together the squares of the abovementioned differences; and
6) on the basis of the result obtained, assign another value to each of the parameters a, b, c, $q_1$, $q_2$, $d_1$, $e_1$, $f_1$, $d_2$, $e_2$, $f_2$, so as to reduce the sum of the squares of the abovementioned differences.

Said processing unit 10 then repeated the abovementioned steps 2) to 6) until said sum of the squares of the differences was minimized and values of the parameters a, b, c, $q_1$, $q_2$, $d_1$, $e_1$, $f_1$, $d_2$, $e_2$, $f_2$ were thus obtained, said values, inserted in the relation (A), generating values of G' which best approximated those obtained experimentally.

The values of the parameters a, b, c, $q_1$, $q_2$, $d_1$, $e_1$, $f_1$, $d_2$, $e_2$, $f_2$ thus determined were then stored in the memory 20 and output (displayed on a computer screen) via the output 40.

These values are shown as follows:

$\Rightarrow$ a = 9.71E+01;
$\Rightarrow$ b = -1.85E+04;
$\Rightarrow$ c = 1.88E+06;
$d_1$ = 8.39E+02;
$\Rightarrow$ $e_1$ = -2.24E+05;
$\Rightarrow$ $f_1$ = 1.48E+07;
$\Rightarrow$ $q_1$ = 1.45E+00;
$\Rightarrow$ $d_2$ = 1.43E+03;
$\Rightarrow$ $e_2$ = -1.51E+05;
$\Rightarrow$ $f_2$ = 4.20E+06;
$\Rightarrow$ $q_2$ = 8.38E+00.

These values were then inserted by said processing unit in the abovementioned relation (A) in order to determine the progression of G' as a function of the torsion $\gamma$ at the temperatures of (-20, -10, 0, 10, 23, 70) $\pm$ 2°C.

The progressions of G' as a function of the torsion $\gamma$ thus determined were then stored in the memory 20 and output (displayed on a computer screen) via the output 40.

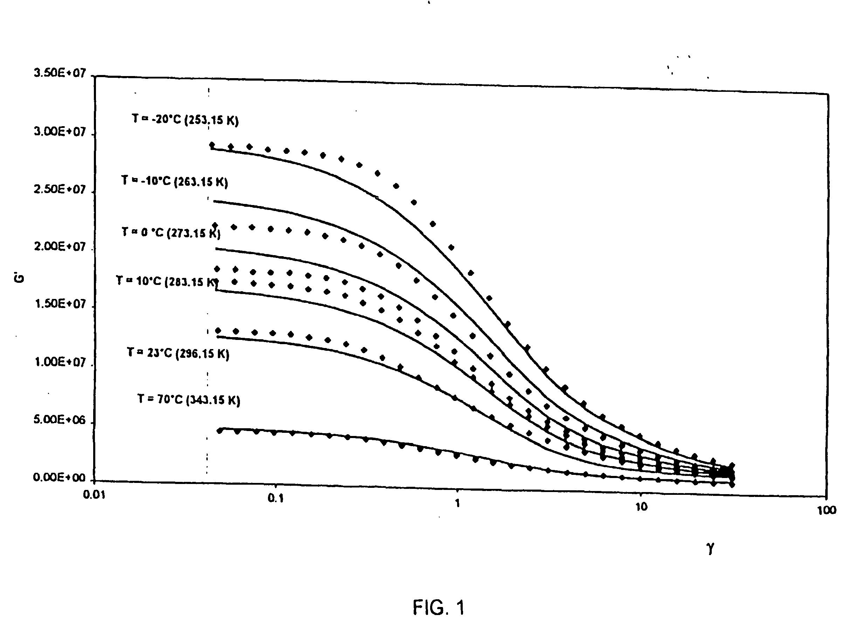

Fig. 1 shows the curves which represent the progression of G' (expressed in Pascal) as a function of $\gamma$ (expressed in %), obtained with the method according to the invention (solid lines) and the corresponding experimental curves which pass through all the points (shown as squares) which represent the values of G' determined experimentally.

As it can be seen, the mean error of the elastic modulus G' calculated using the method according to the invention is equal to about 3% of the mean value measured. In other words, the curve obtained by means of the method according to the invention has a mean deviation, from the curve obtained using the experimental values, of about 3%.

By using the relation (A) and determining a suitable value for each of the parameters a, b, c, $q_1$, $q_2$, $d_1$, $e_1$, $f_1$, $d_2$, $e_2$, $f_2$, the method according to the invention therefore enables one to reproduce accurately the results obtained experimentally.

Moreover, it should be noted that the experimental curves according to Fig. 1 are the result of 29 x 6 values of elastic modulus G' measured as a function of 29 values of the deformation at 6 different temperatures, equal to (NxM) +(N+M)=(29*6)+(29+6)=209 values to be stored. As a result of the invention, therefore, it is possible to reduce the values to be stored for characterization of a dynamic property of a viscoelastic material, from 209 to 11, i.e. to about 5.3% of the initial value.

Even though, in this example, at the 6 temperatures of (-20, -10, 0, 10, 23, 70) $\pm$ 2°C the same values for the deformation $\gamma$ were used, the person skilled in the art will nevertheless easily realize that deformation values which are different from one temperature to another may be used without thereby altering the spirit of the present invention.

Example 2

The same procedure as that used in the preceding Example 1 was used, except that 30 phr (parts by weight per 100 parts of polymeric matrix) of carbon black, type N234, and 30 phr of silica, type VN3, distributed by DEGUSSA and with a surface area equal to 160 $m^2$/g were added to the polymeric composition.

The torsional tests were carried out at the temperatures of (-10, 23, 70) $\pm$ 2°C.

Application of the method according to the invention, as already described above, produced values of the parameters a, b, c, $q_1$, $q_2$, $d_1$, $e_1$, $f_1$, $d_2$, $e_2$, $f_2$ which generate three curves which best approximate the three experimental curves passing through all the points determined experimentally at said temperatures of (-10, 23, 70) $\pm$ 2°C.

The abovementioned values of the parameters a, b, c, $q_1$, $q_2$, $d_1$, $e_1$, $f_1$, $d_2$, $e_2$, $f_2$ were then inserted in the abovementioned relation (A) in order to determine the progression of the elastic modulus G' as a function of the deformation $\gamma$ at the temperatures of 0°C and 10°C at which experimental measurements had not yet been performed.

Subsequently, by way of comparison, experimental measurements of the elastic modulus G' as a function of the deformation $\gamma$ also at the temperatures of 0°C and 10°C were performed.

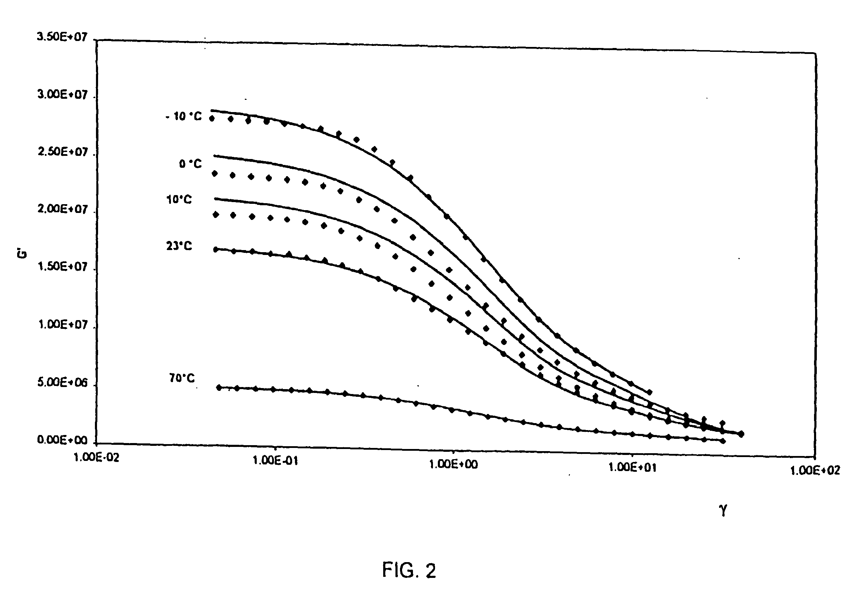

Fig. 2 shows:

* the curves which pass through all the points (shown as squares) which represent the values of G' (expressed in Pascal) as a function of $\gamma$ (expressed in %) determined experimentally at the temperatures of -10, 23 and 70°C;
* the curves representing the progression of G' as a function of $\gamma$, obtained by means of the method according to the invention at the temperatures of - 10, 23 and 70°C (solid lines) ;
* the curves representing the progression of G' as a function of $\gamma$, obtained by means of the method according to the invention at the temperatures of 0 and 10°C (solid lines) ;
* the curves which pass through all the points (shown as squares) which represent the values of G' as a function of $\gamma$, determined experimentally at the temperatures of 0 and 10°C.

Fig. 2 shows that even though the values of the parameters a, b, c, q1, q2, d1, e1, f1, d2, e2, f2 were determined only on the basis of the experimental measurements performed at the temperatures of -10, 23 and 70°C, there is an excellent correlation between the experimental values and the values calculated by means of the relation (A) also in the case of the curves plotted at the temperatures of 0 and 10°C.

Example 3

The same procedure as that used in the preceding Example 1 was used, except for the fact that the viscous modulus G" was measured.

By applying the method according to the invention as described above, the following values for the parameters a, b, c, $q_1$, $q_2$, $d_1$, $e_1$, $f_1$, $d_2$, $e_2$, $f_2$ were obtained:

$\Rightarrow$ a = 292.38;
$\Rightarrow$ b = -28985.6;
$\Rightarrow$ c = 860063.91;
$\Rightarrow$ $d_1$ = -607.41;
$\Rightarrow$ $e_1$ = 75866.44;
$\Rightarrow$ $f_1$ = -2677405.17;
$\Rightarrow$ $q_1$ = 0.7617;
$\Rightarrow$ $d_2$ = 686.74;
$\Rightarrow$ $e_2$ = -94057.14;
$\Rightarrow$ $f_2$ = 4066687.48;
$\Rightarrow$ $q_2$ = 5.272.

These values were then inserted in the abovementioned relation (A) in order to determine the progression of G" as a function of the torsion $\gamma$ at the temperatures of (-20, -10, 0, 10, 23, 70) $\pm$ 2°C.

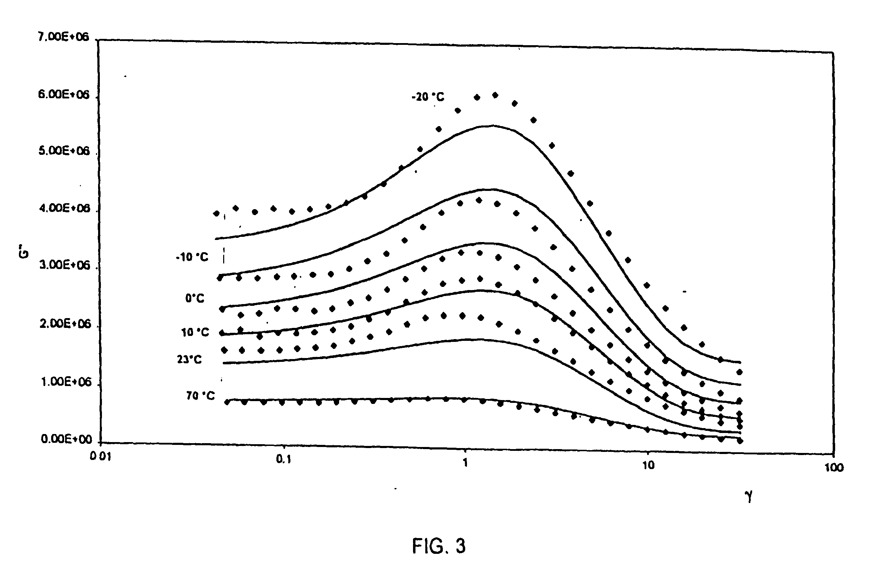

Fig. 3 shows the curves thus obtained (solid lines) which represent the progression of G" (expressed in Pascal) as a function of $\gamma$ (expressed in %) and the corresponding experimental curves which pass through all the points (shown as squares) which represent the values determined experimentally.

In this case also, it will be noted that there is an excellent correlation between the values obtained experimentally and those determined by means of the relation (A) in accordance with the method of the invention.

Example 4

The same procedure as that used in the preceding Example 2 was used, except that the viscous modulus G" as a function of the deformation $\gamma$ at the temperatures of -10, 23 and 70°C was measured.

By applying the method according to the invention, the following values for the parameters $a$, $b$, $c$, $q_1$, $q_2$, $d_1$, $e_1$, $f_1$, $d_2$, $e_2$, $f_2$ were obtained:

$\Rightarrow a = 415.14$;
$\Rightarrow b = -48116.2$;
$\Rightarrow c = 1613000.35$;
$\Rightarrow d_1 = -584.28$;
$\Rightarrow e_1 = 77979.45$;
$\Rightarrow f_1 = -2768211.79$;
$\Rightarrow q_1 = 0.7136$;
$\Rightarrow d_2 = 593.61$;
$\Rightarrow e_2 = -94259.56$;
$\Rightarrow f_2 = 4403028.61$;
$\Rightarrow q_2 = 5.327$.

The abovementioned values of the parameters $a$, $b$, $c$, $q_1$, $q_2$, $d_1$, $e_1$, $f_1$, $d_2$, $e_2$, $f_2$ were then inserted in the abovementioned relation (A) so as to plot the curves for the progression of the viscous modulus G" as a function of the deformation $\gamma$ at the temperatures of 0°C and 10°C at which experimental measurements had not yet been performed.

Subsequently, by way of comparison, experimental measurements of the viscous modulus G" as a function of the deformation $\gamma$ were also performed at the temperatures of 0°C and 10°C.

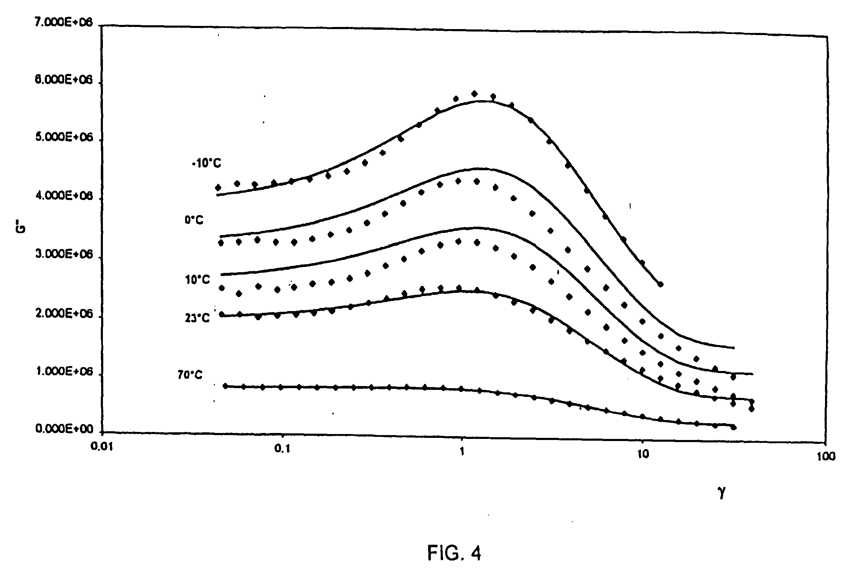

Fig. 4 shows

* the curves which pass through all the points (represented by squares) which represent the values of G'' (expressed in Pascal) as a function of $\gamma$ (expressed in %) determined experimentally at the temperatures of -10, 23 and 70°C;
* the curves representing the progression of G" as a function of $\gamma$, obtained by means of the methods according to the invention at the temperatures of -10, 23 and 70°C (solid lines);
* the curves representing the progression of G" as a function of $\gamma$, obtained by means of the methods according to the invention at the temperatures of 0 and 10°C (solid lines);
* the curves which pass through all the points (shown as squares) which represent the values of G" as a function of $\gamma$, determined experimentally at the temperatures of 0 and 10°C.

In this case also, Fig. 4 shows that despite the fact that the values of the parameters $a$, $b$, $c$, $q_1$, $q_2$, $d_1$, $e_1$, $f_1$, $d_2$, $e_2$, $f_2$ were determined only on the basis of the experimental measurements performed at the temperatures of -10, 23 and 70°C, there is an excellent correlation between experimental values and values calculated by means of the relation (A) also in the case of the curves plotted at the temperatures of 0 and 10°C.

As can be seen from Examples 1 to 4, with the method according to the invention it is possible to reproduce the experimentally measured values of a dynamic parameter P. The minimum deviations which arise between the experimental curves and those determined using the abovementioned relation (A) do not alter at all the reliability of the calculations performed subsequently, on the basis of the calculated values of P', P", P* and tan5 as a function of q, during design of products such as, for example, motor vehicle tyres.

From the above examples it is also obvious that, with the method according to the invention, it is possible to determine the progression of a dynamic parameter P as a function of a deformation q at a temperature $T_w$ at which experimental measurements have not been performed and reduce considerably the number of values which must be stored in order to retain a series of experimental measurements carried out on a viscoelastic material.

**Claims**

1. Method for determining the behaviour of a viscoelastic material at a temperature $T_w$, **characterized in that**:

a) N experimental measurements of a dynamic parameter P, where N $\geq$ 5, as a function of a deformation q at each of at least three temperatures $T_x$, $T_y$, $T_z$ different from $T_w$ are performed;
b) for each of said at least three temperatures $T_x$, $T_y$, $T_z$, the experimental curve which passes through all the points which represent the N values determined experimentally in said step a) is plotted;
c) at least 11 values of said dynamic parameter P and of the associated deformation q distributed along the experimental curves plotted in said step b) at said at least three temperatures $T_x$, $T_y$, $T_z$ are chosen;
d) said at least 11 values are inserted in the relation (A):

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_2}}$$

where

- q is the deformation expressed in %;
- T is the temperature expressed in Kelvin;
- a, b, c are characteristic constants which depend on the type of viscoelastic material and the type of dynamic parameter considered;
- $d_i$, $e_i$, $f_i$ are characteristic constants which depend on the effect of the temperature on the dynamic parameter P and on the ith characteristic deformation;
- $q_i$ is the characteristic deformation at the ith exponential,

so as to determine, for subsequent approximations, values of parameters a, b, c, $q_1$, $q_2$, $d_1$, $e_1$, $f_1$, $d_2$, $e_2$ and $f_2$ of said relation (A) which generate the curves which best approximate said experimental curves plotted in step b); and
e) the temperature $T_w$ and the values of said parameters a, b, c, $q_1$, $q_2$, $d_1$, $e_1$, $f_1$, $d_2$, $e_2$, $f_2$ obtained in the previous step d) are inserted in said relation (A) in order to determine the progression of said dynamic parameter P as a function of the deformation q at the temperature $T_w$.

2. Method according to Claim 1, **characterized in that** said dynamic parameter P is selected from the group comprising the elastic modulus P', the viscous modulus P", the complex modulus P* and the loss factor tanδ of said viscoelastic material.

3. Method according to Claim 1 or 2, **characterized in that**, in the abovementioned step c), at least two values of said dynamic parameter P and the associated deformation q for each of said experimental curves are chosen.

4. Method according to any one of Claims 1 to 3, **characterized in that**, when it is desired to obtain a greater accuracy, the additional parameters required are determined and in place of the summation (I) is performed

$$P(q,T) = a*T^2 + b*T + c + \sum_{i=1}^{\infty}\left(d_i*T^2 + e_i*T + f_i\right)*e^{-\frac{q}{q_i}} \qquad (I)$$

where

- q is the deformation expressed in %;
- T is the temperature expressed in Kelvin;
- a, b, c are characteristic constants which depend on the type of viscoelastic material and the type of dynamic parameter considered;
- $d_i$, $e_i$, $f_i$ are characteristic constants which depend on the effect of the temperature on the dynamic parameter P and on the ith characteristic deformation;
- $q_i$ is the characteristic deformation at the ith exponential.

**5.** Method for improving storage of at least three experimental curves, each obtained from at least N measured experimental values, where N is equal to at least 5, of a dynamic parameter P of a viscoelastic material as a function of a deformation q at one of at least three predetermined temperatures $T_x$, $T_y$, $T_z$, said method being **characterized in that** it comprises the following steps:

a) at least 11 values of said dynamic parameter P and of the associated deformation q distributed along said at least 3 experimental curves obtained in said step b) at said at least three temperatures $T_x$, $T_y$, $T_z$ are chosen;
b) said at least 11 values are inserted in the relation (A):

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_2}}$$

where

- q is the deformation expressed in %;
- T is the temperature expressed in Kelvin;
- a, b, c are characteristic constants which depend on the type of viscoelastic material and the type of dynamic parameter considered;
- $d_i$, $e_i$, $f_i$ are characteristic constants which depend on the effect of the temperature on the dynamic parameter P and on the ith
characteristic deformation;
- $q_i$ is the characteristic deformation at the ith exponential,
so as to determine, for subsequent approximations, values of parameters a, b, c, $q_1$, $q_2$, $d_1$, $e_1$, $f_1$, $d_2$, $e_2$, $f_2$ of said relation (A) which generate the curves which best approximate said at least 3 experimental curves; and

c) the values of said parameters a, b, c, $q_1$, $q_2$, $d_1$, $e_1$, $f_1$, $d_2$, $e_2$, $f_2$ thus obtained are stored.

**6.** Method according to Claim 5, **characterized in that**, when it is desired to obtain greater accuracy, the additional parameters required are determined and the summation (I) is performed:

$$P(q,T) = a*T^2 + b*T + c + \sum_{i=1}^{\infty}\left(d_i*T^2 + e_i*T + f_i\right)*e^{-\frac{q}{q_i}} \qquad (\text{I})$$

where

- q is the deformation expressed in %;
- T is the temperature expressed in Kelvin;
- a, b, c are characteristic constants which depend on the type of viscoelastic material and on the type of dynamic parameter considered;
- $d_i$, $e_i$, $f_i$ are characteristic constants which depend on the effect of the temperature on the dynamic parameter P and on the ith characteristic deformation;
- $q_i$ is the characteristic deformation at the ith exponential.

**7.** Method for defining, from among a plurality of viscoelastic materials, at least one viscoelastic material having a dynamic parameter P which, at a preselected temperature $T_w$ and deformation $q_w$, has a predetermined value $P_w$, said method being **characterized in that** it comprises the steps of:

a) determining, for each of said plurality of viscoelastic materials, the value which said dynamic parameter P assumes at said temperature $T_w$ for said deformation $q_w$ by means of the relation (A.1) :

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_i}}$$

where

- q is the deformation expressed in % ;
- T is the temperature expressed in Kelvin;
- a, b, c are characteristic constants which depend on the type of viscoelastic material and the type of dynamic parameter considered;
- $d_i$, $e_i$, $f_i$ are characteristic constants which depend on the effect of the temperature on the dynamic parameter P and on the ith characteristic deformation;
- $q_i$ is the characteristic deformation at the ith exponential,
and for each of said plurality of viscoelastic materials, the values of the parameters a, b, c, $q_1$, $q_2$, $d_1$, $e_1$, $f_1$, $d_2$, $e_2$, $f_2$ have already been determined beforehand;

b) comparing each of the values of said parameter P thus determined with said value Pw; and

c) defining the viscoelastic materials for which, at the temperature $T_w$ and for the deformation $q_w$, said dynamic parameter P satisfies said value $P_w$.

8. Method according to Claim 7, **characterized in that**, when it is desired to obtain greater accuracy, the summation (I) is used:

$$P(q,T) = a*T^2 + b*T + c + \sum_{i=1}^{\infty}\left(d_i*T^2 + e_i*T + f_i\right)*e^{-\frac{q}{q_i}} \qquad (\text{I})$$

as far as the term which ensures the desired accuracy, where the values of the parameters a, b, c, $d_i$, $e_i$, $f_i$, $q_i$ have already been determined beforehand.

**Patentansprüche**

1. Verfahren zum Bestimmen des Verhaltens eines viskoelastischen Materials bei einer Temperatur $T_w$, **dadurch gekennzeichnet, dass**

a) N experimentelle Messungen eines dynamischen Parameters P als Funktion einer Verformung q bei jeder von wenigstens drei Temperaturen $T_x$, $T_y$, $T_z$ ausgeführt werden, die von $T_w$ verschieden sind, wobei N ≥ 5 ist,
b) für jede der wenigstens drei Temperaturen $T_x$, $T_y$, $T_z$ die experimentelle Kurve aufgetragen wird, die durch alle Punkte geht, die die N Werte darstellen, die experimentell im Schritt a) bestimmt wurden,
c) wenigstens elf Werte des dynamischen Parameters P und der zugeordneten Verformung q gewählt werden, die längs der experimentellen Kurven verteilt sind, die im Schritt b) bei den wenigstens drei Temperaturen $T_x$, $T_y$, $T_z$ aufgetragen wurden,
d) die wenigstens elf Werte in die Gleichung (A) eingesetzt werden:

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_i}}$$

wobei

- q die in Prozent ausgedrückte Verformung,
- T die in Kelvin ausgedrückte Temperatur,
- a, b, c charakteristische Konstanten, die von der Art des viskoelastischen Materials und von der Art des in Betracht gezogenen dynamischen Parameters abhängen,

- $d_i$, $e_i$, $f_i$ charakteristische Konstanten, die von der Wirkung der Temperatur auf den dynamischen Parameter P und von der auf die i-ten charakteristischen Verformung abhängen, und
- $q_i$ die charakteristische Verformung bei der i-ten Exponentialgröße sind,

um für darauffolgende Approximierungen Werte der Parameter a, b, c, $q_1$, $q_2$, $d_1$, $e_1$, $f_1$, $d_2$, $e_2$ und $f_2$ der Gleichung (A) zu bestimmen, die die Kurven erzeugen, die die im Schritt b) aufgetragenen experimentellen Kurven am besten approximieren, und

e) die Temperatur $T_w$ und die Werte der Parameter a, b, c, $q_1$, $q_2$, $d_1$, $e_1$, $f_1$, $d_2$, $e_2$, $f_2$, die bei dem vorherigen Schritt d) erhalten wurden, in die Gleichung (A) eingesetzt werden, um den Verlauf des dynamischen Parameters P als Funktion der Verformung q bei der Temperatur $T_w$ zu bestimmen.

2. Verfahren nach Anspruch 1, **dadurch gekennzeichnet, dass** der dynamische Parameter P aus der Gruppe ausgewählt wird, die den Elastizitätsmodul P', den Viskositätsmodul P'', den komplexen Modul P* und den Verlustfaktor $\tan\delta$ des viskoelastischen Materials aufweist.

3. Verfahren nach Anspruch 1 oder 2, **dadurch gekennzeichnet, dass** bei dem vorstehenden Schritt c) wenigstens zwei Werte des dynamischen Parameters P und der zugeordneten Verformung q für jede der experimentellen Kurven gewählt werden.

4. Verfahren nach einem der Ansprüche 1 bis 3, **dadurch gekennzeichnet, dass**, wenn man eine größere Genauigkeit erhalten möchte, die erforderlichen zusätzlichen Parameter bestimmt werden und die Summierung (I) ausgeführt wird

$$P(q,T) = a*T^2 + b*T + c + \sum_{i=1}^{\infty}\left(d_i*T^2 + e_i*T + f_i\right)*e^{-\frac{q}{q_i}} \qquad (I)$$

wobei

- q die in Prozent ausgedrückte Verformung,
- T die in Kelvin ausgedrückte Temperatur,
- a, b, c charakteristische Konstanten, die von der Art des viskoelastischen Materials und von der Art des in Betracht gezogenen dynamischen Parameters abhängen,
- $d_i$, $e_i$, $f_i$ charakteristische Konstanten, die von der Wirkung der Temperatur auf den dynamischen Parameter P und von der i-ten charakteristischen Verformung abhängen, und
- $q_i$ die charakteristische Verformung bei der i-ten Exponentialgröße sind.

5. Verfahren zum Verbessern der Speicherung von wenigstens drei experimentellen Kurven, von denen jede aus wenigstens N gemessenen experimentellen Werten eines dynamischen Parameters P eines viskoelastischen Materials als Funktion einer Verformung q bei einer von wenigstens drei vorgegebenen Temperaturen $T_x$, $T_y$, $T_z$ erhalten wird, wobei N gleich wenigstens 5 ist, wobei das Verfahren **dadurch gekennzeichnet ist, daß** es die folgenden Schritte aufweist:

a) es werden wenigstens elf Werte des dynamischen Parameters P und der zugeordneten Verformung q gewählt, die längs der wenigstens drei experimentellen Kurven verteilt sind, die im Schritt b) bei den wenigstens drei Temperaturen $T_x$, $T_y$, $T_z$ erhalten wurden,
b) es werden die wenigstens elf Werte in die Gleichung (A) eingesetzt,

$$P(q,T) = a*T^2 + b*T + c + (d_1*T^2 + e_1*T + f_1)*e^{\frac{q}{q_1}} + (d_2*T^2 + e_2*T + f_2)*e^{\frac{q}{q_2}}$$

wobei

- q die in Prozent ausgedrückte Verformung,
- T die in Kelvin ausgedrückte Temperatur,
- a, b, c charakteristische Konstanten, die von der Art des viskoelastischen Materials und von der Art des in Betracht gezogenen dynamischen Parameters abhängen,

- di, $e_i$, $f_i$ charakteristische Konstanten, die von der Wirkung der Temperatur auf den dynamischen Parameter P und von der i-ten charakteristischen Verformung abhängen, und
- $q_i$ die charakteristische Verformung bei der i-ten Exponentialgröße sind,

um für darauffolgende Approximierungen Werte der Parameter a, b, c, $q_1$, $q_2$, $d_1$, $e_1$, $f_1$, $d_2$, $e_2$ und $f_2$ der Gleichung (A) zu bestimmen, die die Kurven erzeugen, die die wenigstens drei experimentellen Kurven am besten approximieren, und

c) es werden die so erhaltenen Werte der Parameter a, b, c, $q_1$, $q_2$, $d_1$, $e_1$, $f_1$, $d_2$, $e_2$, $f_2$ gespeichert.

6. Verfahren nach Anspruch 5, **dadurch gekennzeichnet, dass**, wenn man eine größere Genauigkeit erhalten möchte, die zusätzlichen erforderlichen Parameter bestimmt werden und die Summierung (I) ausgeführt wird:

$$P(q,T) = a*T^2 + b*T + c + \sum_{i=1}^{\infty}\left(d_i*T^2 + e_i*T + f_i\right)*e^{-\frac{q}{q_i}} \qquad (\mathrm{I})$$

wobei

- q die in Prozent ausgedrückte Verformung,
- T die in Kelvin ausgedrückte Temperatur,
- a, b, c charakteristische Konstanten, die von der Art des viskoelastischen Materials und der Art des in Betracht gezogenen dynamischen Parameters abhängen,
- di, $e_i$, $f_i$ charakteristische Konstanten, die von der Wirkung der Temperatur auf den dynamischen Parameter P und von der i-ten charakteristischen Verformung abhängen, und
$q_i$ die charakteristische Verformung bei der i-ten Exponentialgröße sind.

7. Verfahren zum Bestimmen aus einer Vielzahl von viskoelastischen Materialien wenigstens eines viskoelastischen Materials, das einen dynamischen Parameter P hat, der bei einer vorher ausgewählten Temperatur $T_w$ und einer Verformung $q_w$ einen vorgegebenen Wert $P_w$ hat, wobei das Verfahren **dadurch gekennzeichnet ist, dass** es die Schritte aufweist:

a) Bestimmen mit Hilfe der Gleichung (A.1) des Wertes für jedes viskoelastische Material aus der Vielzahl von viskoelastischen Materialien, den der dynamische Parameter P bei der Temperatur $T_w$ für die Verformung $q_w$ annimmt,

$$P(q,T) = a*T^2 + b*T + c + (d_1 * T^2 + e_1* T + f_1)*\ e^{\frac{q}{q_1}} + (d_2 * T^2 + e_2 * T + f_2)*\ e^{\frac{q}{q_2}}$$

wobei

- q die in Prozent ausgedrückte Verformung,
- T die in Kelvin ausgedrückte Temperatur,
- a, b, c charakteristische Konstanten, die von der Art des viskoelastischen Materials und von der Art des in Betracht gezogenen dynamischen Parameters abhängen,
- $d_i$, $e_i$, $f_i$ charakteristische Konstanten, die von der Wirkung der Temperatur auf den dynamischen Parameter P und von der i-ten charakteristischen Verformung abhängen,
- $q_i$ die charakteristische Verformung bei der i-ten Exponentialgröße sind, und
- für jedes viskoelastische Material der Vielzahl von viskoelastischen Materialien die Werte der Parameter a, b, c, $q_1$, $q_2$, $d_1$, $e_1$, $f_1$, $d_2$, $e_2$, $f_2$ bereits vorher bestimmt worden sind,

b) Vergleichen eines jeden der Werte des Parameters P, der so mit dem Wert $P_w$ bestimmt wurde, und
c) Bestimmen der viskoelastischen Materialien, für die bei der Temperatur $T_w$ und für die Verformung $q_w$ der dynamische Parameter P dem Wert $P_w$ genügt.

8. Verfahren nach Anspruch 7, **dadurch gekennzeichnet, dass**, wenn man eine größere Genauigkeit erhalten möchte, die Summierung (I) verwendet wird

$$P(q,T) = a*T^2 + b*T + c + \sum_{i=1}^{\infty}\left(d_i*T^2 + e_i*T + f_i\right)*e^{-\frac{q}{q_i}} \qquad (\text{I})$$

soweit für den Ausdruck, der die gewünschte Genauigkeit gewährleistet, die Werte der Parameter a, b, c, $d_i$, $e_i$, $f_i$, $q_i$ bereits vorher bestimmt worden sind.

## Revendications

1. Procédé de détermination du comportement d'un matériau viscoélastique à une température $T_w$, **caractérisé en ce que**:

    a) on effectue N mesures expérimentales d'un paramètre dynamique P, où $N \geq 5$, en fonction d'une déformation q à chacune d'au moins trois températures $T_x$, $T_y$, $T_z$ différentes de $T_w$;
    b) pour chacune desdites au moins trois températures $T_x$, $T_y$, $T_z$, on trace la courbe expérimentale qui passe par tous les points qui représentent les N valeurs déterminées expérimentalement à ladite étape a);
    c) on choisit au moins 11 valeurs dudit paramètre dynamique P et de la déformation q associée réparties le long des courbes expérimentales tracées à ladite étape b) auxdites au moins trois températures $T_x$, $T_y$, $T_z$;
    d) on insère lesdites au moins 11 valeurs dans la relation (A):

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_2}}$$

    dans laquelle:

    - q est la déformation exprimée en %;
    - T est la température exprimée en kelvins;
    - a, b, c sont des constantes caractéristiques qui dépendent du type de matériau viscoélastique et du type de paramètre dynamique considéré;
    - $d_i$, $e_i$, $f_i$ sont des constantes caractéristiques qui dépendent de l'effet de la température sur le paramètre dynamique P et sur la $i^{\text{ème}}$ déformation caractéristique;
    - $q_i$ est la déformation caractéristique dans la $i^{\text{ème}}$ exponentielle,

    de manière à déterminer, pour des approximations suivantes, des valeurs des paramètres a, b, c, $q_1$, $q_2$, $d_1$, $e_1$, $f_1$, $d_2$, $e_2$ et $f_2$ de ladite relation (A) qui génèrent les courbes qui approchent le mieux lesdites courbes expérimentales tracées à l'étape b); et
    e) on insère la température $T_w$ et les valeurs desdits paramètres a, b, c, $q_1$, $q_2$, $d_1$, $e_1$, $f_1$, $d_2$, $e_2$, $f_2$ obtenues à l'étape d) précédente dans ladite relation (A) afin de déterminer la progression dudit paramètre dynamique P en fonction de la déformation q à la température $T_w$.

2. Procédé selon la revendication 1, **caractérisé en ce que** ledit paramètre dynamique P est choisi dans le groupe comprenant le module d'élasticité P', le module de viscosité P'', le module complexe P* et le facteur de perte tg$\delta$ dudit matériau viscoélastique.

3. Procédé selon la revendication 1 ou 2, **caractérisé en ce que**, à ladite étape c), on choisit au moins deux valeurs dudit paramètre dynamique P et de la déformation q associée pour chacune desdites courbes expérimentales.

4. Procédé selon l'une quelconque des revendications 1 à 3, **caractérisé en ce que**, lorsqu'on souhaite obtenir une plus grande précision, on détermine les paramètres supplémentaires requis et on effectue la somme (I):

$$P(q,T) = a*T^2 + b*T + c + \sum_{i=1}^{\infty}\left(d_i*T^2 + e_i*T + f_i\right)*e^{-\frac{q}{q_i}} \qquad (\text{I})$$

dans laquelle :

- q est la déformation exprimée en %;
- T est la température exprimée en kelvins;
- a, b, c sont des constantes caractéristiques qui dépendent du type de matériau viscoélastique et du type de paramètre dynamique considéré;
- $d_i$, $e_i$, $f_i$ sont des constantes caractéristiques qui dépendent de l'effet de la température sur le paramètre dynamique P et sur la $i^{ème}$ déformation caractéristique;
- $q_i$ est la déformation caractéristique dans la $i^{ème}$ exponentielle.

5. Procédé pour améliorer le stockage d'au moins trois courbes expérimentales, obtenues chacune à partir d'au moins N valeurs expérimentales mesurées, où N est égal à au moins 5, d'un paramètre dynamique P d'un matériau viscoélastique en fonction d'une déformation q à l'une d'au moins trois températures $T_x$, $T_y$, $T_z$ prédéterminées, ledit procédé étant **caractérisé en ce qu'**il comprend les étapes suivantes:

a) on choisit au moins 11 valeurs dudit paramètre dynamique P et de la déformation q associée réparties le long desdites au moins 3 courbes expérimentales obtenues à ladite étape b) auxdites au moins trois températures $T_x$, $T_y$, $T_z$;
b) on insère lesdites au moins 11 valeurs dans la relation (A):

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_2}}$$

dans laquelle:

q est la déformation exprimée en %;
- T est la température exprimée en kelvins;
- a, b, c sont des constantes caractéristiques qui dépendent du type de matériau viscoélastique et du type de paramètre dynamique considéré;
- $d_i$, $e_i$, $f_i$ sont des constantes caractéristiques qui dépendent de l'effet de la température sur le paramètre dynamique P et sur la $i^{ème}$ déformation caractéristique;
- $q_i$ est la déformation caractéristique dans la $i^{ème}$ exponentielle,
de manière à déterminer, pour des approximations suivantes, des valeurs des paramètres a, b, c, $q_1$, $q_2$, $d_1$, $e_1$, $f_1$, $d_2$, $e_2$, $f_2$ de ladite relation (A) qui génèrent les courbes qui approchent le mieux lesdites au moins 3 courbes expérimentales; et
c) on stocke les valeurs desdits paramètres a, b, c, $q_1$, $q_2$, $d_1$, $e_1$, $f_1$, $d_2$, $e_2$, $f_2$ ainsi obtenues.

6. Procédé selon la revendication 5, **caractérisé en ce que**, lorsqu'on souhaite obtenir une plus grande précision, on détermine les paramètres supplémentaires requis et on effectue la somme (I):

$$P(q,T) = a*T^2 + b*T + c + \sum_{i=1}^{\infty}\left(d_i*T^2 + e_i*T + f_i\right)*e^{-\frac{q}{q_i}} \qquad (I)$$

dans laquelle :

- q est la déformation exprimée en %;
- T est la température exprimée en kelvins;
- a, b, c sont des constantes caractéristiques qui dépendent du type de matériau viscoélastique et du type de paramètre dynamique considéré;
- di, $e_i$, $f_i$ sont des constantes caractéristiques qui dépendent de l'effet de la température sur le paramètre dynamique P et sur la $i^{ème}$ déformation caractéristique;
- $q_i$ est la déformation caractéristique dans la $i^{ème}$ exponentielle.

7. Procédé pour définir, parmi une pluralité de matériaux viscoélastiques, au moins un matériau viscoélastique ayant un paramètre dynamique P qui, à une température $T_w$ et une déformation $q_w$ présélectionnées, prend une valeur

$P_w$ prédéterminée, ledit procédé étant **caractérisé en ce qu'**il comprend les étapes consistant à:

a) déterminer, pour chacun desdits matériaux viscoélastiques, la valeur que prend ledit paramètre dynamique P à ladite température $T_w$ pour ladite déformation $q_w$ au moyen de la relation (A.1):

$$P(q,T) = a*T^2 + b*T + c + \left(d_1*T^2 + e_1*T + f_1\right)*e^{-\frac{q}{q_1}} + \left(d_2*T^2 + e_2*T + f_2\right)*e^{-\frac{q}{q_2}}$$

dans laquelle:

- q est la déformation exprimée en %;
- T est la température exprimée en kelvins;
- a, b, c sont des constantes caractéristiques qui dépendent du type de matériau viscoélastique et du type de paramètre dynamique considéré;
- $d_i$, $e_i$, $f_i$ sont des constantes caractéristiques qui dépendent de l'effet de la température sur le paramètre dynamique P et sur la $i^{ième}$ déformation caractéristique;
- $q_i$ est la déformation caractéristique dans la $i^{ième}$ exponentielle,
et pour chacun desdits matériaux viscoélastiques, les valeurs des paramètres a, b, c, $q_1$, $q_2$, $d_1$, $e_1$, $f_1$, $d_2$, $e_2$, $f_2$ ont déjà été déterminées au préalable;

b) comparer chacune des valeurs dudit paramètre P ainsi déterminées à ladite valeur $P_w$; et
c) définir les matériaux viscoélastiques pour lesquels, à la température $T_w$ et pour la déformation $q_w$, ledit paramètre dynamique P prend ladite valeur $P_w$.

8. Procédé selon la revendication 7, **caractérisé en ce que**, lorsqu'on souhaite obtenir une plus grande précision, on utilise la somme (I):

$$P(q,T) = a*T^2 + b*T + c + \sum_{i=1}^{\infty}\left(d_i*T^2 + e_i*T + f_i\right)*e^{-\frac{q}{q_i}} \qquad (I)$$

jusqu'au terme qui assure la précision voulue, dans laquelle les valeurs des paramètres a, b, c, $d_i$, $e_i$, $f_i$, $q_i$ ont déjà été déterminées au préalable.

FIG. 1

FIG. 2

FIG. 3

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4667519 A

- US 5452614 A